# EUROPÄISCHE PATENTSCHRIFT

(11) **EP 1 682 474 B1**
(45) Veröffentlichungstag und Bekanntmachung des Hinweises auf die Patenterteilung: **14.01.2015**
(21) Anmeldenummer: 04790480.0
(22) Anmeldetag: 15.10.2004
(51) Int. Cl.: C07C 45/35, C07C 47/22, B01J 23/887, B01J 23/94, B01J 38/12

(54) **VERFAHREN ZUM LANGZEITBETRIEB EINER HETEROGEN KATALYSIERTEN GASPHASENPARTIALOXIDATION VON PROPEN ZU ACROLEIN**
METHOD FOR LONG TERM OPERATION OF A HETEROGENEOUSLY CATALYSED GAS PHASE PARTIAL OXIDATION OF PROPENE IN ORDER TO FORM ACROLEIN
PROCEDE D'UTILISATION A LONG TERME D'UNE OXYDATION PARTIELLE EN PHASE GAZEUSE DE PROPENE EN ACROLEINE CATALYSEE DE FAÇON HETEROGENE

(30) Priorität: 29.10.2003 DE 10350812; 29.10.2003 US 515114 P
(43) Veröffentlichungstag der Anmeldung: 26.07.2006
(73) Patentinhaber: BASF SE, 67056 Ludwigshafen (DE)
(72) Erfinder: DIETERLE, Martin, 68167 Mannheim (DE); PETZOLDT, Jochen, 67273 Weisenheim am Berg (DE); MÜLLER-ENGEL, Klaus, Joachim, 76297 Stutensee (DE)
(86) Internationale Anmeldenummer: PCT/EP2004/011638
(87) Internationale Veröffentlichungsnummer: WO 2005/047224

(56) Entgegenhaltungen:
- EP-A- 0 169 449
- EP-A- 0 339 119
- EP-A- 0 614 872
- US-B1- 6 346 646

## Beschreibung

Vorliegende Erfindung betrifft ein Verfahren zum Langzeitbetrieb einer heterogen katalysierten Gasphasenpartialoxidation von Propen zu Acrolein, bei dem man ein Propen, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Molybdän und/oder Wolfram sowie wenigstens eines der Elemente Wismut, Tellur, Antimon, Zinn und Kupfer enthält, und bei dem man, um der Deaktivierung des Katalysatorfestbetts entgegenzuwirken, die Temperatur des Katalysatorfestbetts über die Zeit erhöht.

Acrolein bildet ein reaktives Monomer, das insbesondere als Zwischenprodukt, z.B. bei der Herstellung von Acrylsäure durch zweistufige heterogen katalysierte partielle Gasphasenoxidation von Propen, von Bedeutung ist. Acrylsäure ist als solche oder in Form ihrer Alkylester z.B. zur Herstellung von Polymerisaten, die u.a. als Klebstoffe oder Wasser absorbierende Materialien Verwendung finden können, geeignet.

Es ist bekannt, Acrolein großtechnisch durch ein Verfahren der heterogen katalysierten Gasphasenpartialoxidation von Propen zu Acrolein, bei dem man ein Propen, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Molybdän und/oder Wolfram sowie wenigstens eines der Elemente Wismut, Tellur, Antimon, Zinn und/oder Kupfer enthält, herzustellen (vgl. z.B. EP-A 990636, EP-A 1106598, EP-A 169449, EP-A 700714, DE-A 3300044 und DE-A 19948623).

Es ist auch bekannt, dass ein solches Verfahren der heterogen katalysierten Gasphasenpartialoxidation von Propen zu Acrolein an ein und demselben Katalysatorfestbett im wesentlichen kontinuierlich über längere Zeiträume betrieben werden kann. Allerdings verliert das Katalysatorfestbett dabei im Verlauf der Betriebszeit an Qualität. In der Regel verschlechtern sich sowohl seine Aktivität als auch die Selektivität der Zielproduktbildung (als Zielprodukt wird in dieser Schrift die Summe aus Acrolein und gegebenenfalls als Nebenwertprodukt gebildeter Acrylsäure verstanden).

Um das Katalysatorfestbett, dessen Fertigung und Austausch vergleichsweise aufwändig und kostspielig sind, trotzdem möglichst lange in einem damit beschickten Reaktor betreiben zu können, wird im Stand der Technik auf unterschiedlichste Art und Weise versucht, dem Alterungsprozeß des Katalysatorfestbetts entgegenzuwirken.

Die EP-A 990636 (z. B. Seite 8, Zeilen 13 und 15) und die EP-A 1106598 (z. B. Seite 13, Zeilen 43 bis 45) schlagen vor, die Minderung der Qualität des Katalysatorfestbetts dadurch weitgehend zu kompensieren, dass im Verlauf der Betriebszeit, unter ansonsten weitgehend gleichbleibenden Betriebsbedingungen, die Temperatur des Katalysatorfestbetts nach und nach erhöht wird, um den Propenumsatz bei einmaligem Durchgang das Reaktionsgasgemischs durch das Katalysatorfestbett im wesentlichen beizubehalten.

EP 0 339 119 A1 offenbart ein Verfahren zur Reaktivierung eines Katalysators, der bei der Herstellung eines ungesättigten Aldehyds als Hauptprodukt durch katalytische Dampfphasenoxidationsreaktion von Propylen, Isobutylen oder tertiärem Butanol verwendet worden ist, wobei der Katalysator ein katalytisch aktives Oxid aus Molybdän, Bismuth und Eisen mit einem Mo : Fe-Atomverhältnis von 12 : mindestens 0,1, enthält. Das Verfahren umfasst die Wärmebehandlung des gebrauchten Katalysators bei einer Temperatur von 300 bis 500 °C in Gegenwart eines strömenden Oxidationsgases enthaltend molekularen Sauerstoff und Dampf. Die Behandlung erfolgt erst nach über einem Jahr des kontinuierlichen Betriebes.

US 6,346,646 B1 offenbart ein Verfahren zur Herstellung von Methacrolein und Methacrylsäure durch katalytische Oxidation von Isobutylen oder tertiär-Butanol in Gegenwart eines Katalysators, umfassend ein Mischoxid, enthaltend Molybdän, Bismut und Eisen als wesentliche Komponenten. Eine Reaktivierungsbehandlung des Katalysators wird erst nach über einem Jahr des kontinuierlichen Betriebes durchgeführt.

Unter der Temperatur des Katalysatorfestbetts wird dabei die Temperatur des Katalysatorfestbetts bei Ausübung des Partialoxidationsverfahrens jedoch in fiktive Abwesenheit einer chemischen Reaktion (d. h., ohne den Einfluss der Reaktionswärme) verstanden. Dies soll auch in dieser Schrift gelten. Unter effektiver Temperatur des Katalysatorfestbetts soll in dieser Schrift dagegen die tatsächliche Temperatur des Katalysatorfestbetts unter Einbezug der Reaktionswärme der Partialoxidation verstanden werden. Ist die Temperatur des Katalysatorfestbetts längs des Katalysatorfestbetts nicht konstant (z. B. im Fall von mehreren Temperaturzonen), so meint der Begriff Temperatur des Katalysatorfestbetts in dieser Schrift den (Zahlen)mittelwert der Temperatur entlang des Katalysatorfestbetts.

Von Bedeutung im vorgenannten Zusammenhang ist, dass die Temperatur des Reaktionsgasgemischs (und damit auch die effektive Temperatur des Katalysatorfestbetts) beim Durchgang durch das Katalysatorfestbett einen Höchstwert (den sogenannten Heißpunktwert) durchläuft. Die Differenz zwischen Heißpunktwert und der Temperatur des Katalysatorfestbetts an der Stelle des Heißpunktwertes wird als Heißpunktausdeh nung bezeichnet.

Nachteilig an der in der EP-A 990 636 sowie in der EP-A 1 106 598 empfohlenen Verfahrensweise ist, dass sich mit zunehmender Erhöhung der Temperatur des Katalysatorfestbetts sein Alterungsprozess beschleunigt (bestimmte Bewegungsprozesse innerhalb der Katalysatoren, die zur Alterung beitragen, laufen z. B. schneller ab). Dies in der Regel vor allem auch deshalb, weil die Heißpunktausdehnung mit einer Erhöhung der Temperatur des Katalysatorfestbetts meist noch stärker zunimmt als die Temperatur des Katalysatorfestbetts selbst (vgl. z. B. Seite 12, Zeilen 45 bis 48 der EPA 1 106 598 und Seite 8, Zeilen 11 bis 15 der EP-A 990 636). Die effektive Temperatur des Katalysatorfestbetts nimmt im Heißpunktbereich daher meist überproportional zu, was die Alterung des Katalysatorfestbetts zusätzlich fördert.

Bei Erreichen eines Höchstwerts der Temperatur des Katalysatorfestbetts wird daher das Katalysatorfestbett üblicherweise vollständig ausgetauscht.

Nachteilig an einem solchen Komplettaustausch ist jedoch, dass er vergleichsweise aufwendig ist. Das Verfahren der Acrylsäureherstellung muss für längere Zeit unterbrochen werden und die Kosten der Katalysatorherstellung sind ebenfalls erheblich.

Erwünscht sind daher Betriebsweisen für Verfahren einer heterogen katalysierten Gasphasenpartialoxidation von Propen zu Acrolein, die dabei behilflich sind, die Standzeit des Katalysatorfestbetts im Reaktor weitestgehend in die Länge zu ziehen.

Die DE-A 10232748 empfiehlt diesbezüglich, anstatt das Katalysatorfestbett vollständig auszutauschen, nur eine Teilmenge desselben durch eine frische Katalysatorbeschickung zu ersetzen.

Nachteilig an diesem Vorschlag ist, dass auch mit einem Teilwechsel des Katalysatorfestbetts bereits ein nennenswerter Aufwand einhergeht.

Die EP-A 169449 empfiehlt, die Standzeit des Katalysatorfestbetts dadurch zu verlängern, dass man nach mehrjähriger Betriebsdauer des Katalysatorfestbetts, mit der Erhöhungen der Temperatur desselben von 15°C und mehr einhergehen, das Verfahren der Partialoxidation unterbricht, und bei Katalysatorfestbetttemperaturen von 380 bis 540°C durch selbiges ein im wesentlichen aus Luft bestehendes Gas führt und anschließend die Partialoxidation fortsetzt. Die EP-A 339119 empfiehlt bei analoger Verfahrensweise die Anwendung eines Sauerstoff und Wasserdampf enthaltenden Gases.

In diesem Zusammenhang sollen in dieser Schrift als Inertgase in einem Gasgemisch, das unter bestimmten Bedingungen durch das Katalysatorfestbett geführt wird, solche Gase verstanden werden, die bei der Durchführung durch das Katalysatorfestbett zu wenigstens 95 mol-%, bevorzugt zu wenigstens 98 mol-%, ganz besonders bevorzugt zu wenigstens 99 mol-% oder 99,5 mol-% unverändert erhalten bleiben. Das erfindungsgemäß einzusetzende Gasgemisch G betreffend soll Wasserdampf nicht unter dem Begriff Inertgas subsummieren. Nachteilig an der Verfahrensweise der EP-A 169449 ist jedoch, das bis zum Zeitpunkt der Unterbrechung die Alterung des Katalysatorfestbetts ungebremst fortschreitet und gefördert wird. Das gleich gilt auch für die EP-A 614872.

Wünschenswert wäre insbesondere ein Verfahren zum Langzeitbetrieb einer heterogen katalysierten Gasphasenpartialoxidation von Propen zu Acrolein, bei dem der Katalysatoralterung auf eine Art und Weise entgegengewirkt wird, mit der die Ausprägung der Heißpunktausdehnung über die Zeit geringer ist als bei den Verfahren des Standes der Technik.

Demgemäß wurde ein Verfahren zum Langzeitbetrieb einer heterogen katalysierten Gasphasenpartialoxidation von Propen zu Acrolein, bei dem man ein Propen, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Molybdän und/oder Wolfram sowie wenigstens eines der Elemente Wismut, Tellur, Antimon, Zinn und Kupfer enthält, und bei dem man, um der Deaktivierung des Katalysatorfestbetts entgegenzuwirken, die Temperatur des Katalysatorfestbetts über die Zeit erhöht, gefunden, das dadurch gekennzeichnet ist, dass man die Gasphasenpartialoxidation wenigstens einmal pro Kalenderquartal unterbricht und bei einer Temperatur des Katalysatorfestbetts von 250°C bis 550°C (vorzugsweise 300°C bis 500°C, besonders bevorzugt 350°C bis 450°C bzw. 300 bis 400°C bzw. 300 bis 360°C) ein aus molekularem Sauerstoff, Inertgas und gegebenenfalls Wasserdampf bestehendes Gasgemisch G durch das Katalysatorfestbett führt.

Es überrascht, dass bei Anwendung des erfindungsgemäßen Verfahrens ein Langzeitbetrieb einer heterogen katalysierten Gasphasenpartialoxidation von Propen zu Acrolein möglich ist, mit dem die Ausprägung der Heißpunktausdehnung über die Zeit geringer ist als bei den Verfahren des Standes der Technik. In günstigen Fällen bleibt die Ausprägung der Heißpunktausdehnung über die Zeit konstant oder verhält sich sogar abnehmend. Außerdem bleibt die Selektivität der Zielproduktbildung über die Zeit meist weitgehend konstant und nimmt in günstigen Fällen sogar zu.

Erfindungsgemäß wird man die Gasphasenpartialoxidation wenigstens einmal pro Kalenderquartal und bevorzugt wenigstens einmal pro zwei aufeinanderfolgende Kalendermonate und am besten wenigstens einmal pro Kalendermonat unterbrechen und bei einer Temperatur des Katalysatorfestbetts von 250 bis 550°C ein aus molekularem Sauerstoff, Inertgas und gegebenenfalls Wasserdampf bestehendes Gasgemisch G durch das Katalysatorfestbett führen.

In der Regel wird man die Gasphasenpartialoxidation jedoch wenigstens eine Kalenderwoche kontinuierlich betreiben, bevor sie erfindungsgemäß unterbrochen wird.

In anderen Worten ausgedrückt wird man beim erfindungsgemäßen Verfahren die Durchführung des Gasgemischs G durch das Katalysatorfestbett bei einer Temperatur des Katalysatorfestbetts von 250 bis 550°C wenigstens einmal innerhalb von 2000, oder 1500, oder 1000, oder 500 Betriebsstunden der Partialoxidation durchführen. Eine häufige Durchführung des erfindungsgemäßen Verfahrens wirkt sich vorteilhaft aus.

Erfindungsgemäß günstig wird man die Temperatur des Katalysatorfestbetts während der Durchführung des Gasgemischs G bei der Durchführung des erfindungsgemäßen Verfahrens auf einem Wert T_{G} halten, der im wesentlichen derjenigen Temperatur T_{V} des Katalysatorfestbetts entspricht, bei der die Partialoxidation betrieben wurde, bevor sie unterbrochen wurde, um das Gasgemisch G erfindungsgemäß durch das Katalysatorfestbett zu führen.

D.h., erfindungsgemäß vorteilhaft gilt T_{G} = T_{V} ± 50°C bzw. T_{G} = T_{V} ± 20°C und ganz besonders vorteilhaft ist T_{G} = Tv.

Normalerweise wird T_{V} im Bereich von 250 bis 450°C, häufig im Bereich von 300 bis 400°C liegen.

Die Zeitdauer t_{G} während der beim erfindungsgemäßen Verfahren das Gasgemisch G durch das Katalysatorfestbett zu führen ist, wird in der Regel wenigstens 2 h, häufig 6 h bis 120 h, vielfach 12 h bis 72 h und oft 20 h bis 40 h betragen. Sie kann jedoch auch 10 Tage und mehr betragen. Üblicherweise wird man die Zeitdauer t_{G} so lange bemessen, bis der Sauerstoffgehalt des Gasgemischs G beim Austritt aus dem Katalysatorfestbett sich vom Sauerstoffgehalt des Gasgemischs G beim Eintritt in das Katalysatorfestbett nicht mehr unterscheidet. In der Regel wird ein kleiner Sauerstoffgehalt des Gasgemischs G eine längere Zeitdauer t_{G} bedingen. Erhöhte Sauerstoffgehalte im Gasgemisch G sind erfindungsgemäß vorteilhaft.

In anwendungstechnisch zweckmäßiger Weise wird das Gasgemisch G beim erfindungsgemäßen Verfahren wenigstens 1 Vol.-% bzw. wenigstens 2 Vol.-%, vorzugsweise wenigstens 3 Vol.-% und besonders bevorzugt wenigstens 4 Vol.-% Sauerstoff enthalten. In der Regel wird der Sauerstoffgehalt des Gasgemischs G jedoch ≤ 21 Vol.-% betragen. D.h., ein mögliches Gasgemisch G ist Luft. Ein anderes mögliches Gasgemisch G ist Magerluft. Dabei handelt es sich um an Sauerstoff entreicherte Luft.

Erfindungsgemäß vorteilhaft ist Magerluft, die aus 3 bis 10, vorzugsweise 4 bis 6 Vol.-% Sauerstoff und als Restmenge aus molekularem Stickstoff besteht. Erfindungsgemäß zweckmäßig enthält das Gasgemisch G im wesentlichen keinen Wasserdampf. Das erfindungsgemäß zu verwendende Gasgemisch G kann jedoch bis zu 0,1 Vol.-%, oder bis zu 0,5 Vol.-%, oder bis zu 1 Vol.-% an Wasserdampf enthalten. Normalerweise liegt der Wasserdampfgehalt des Gasgemischs G bei ≤ 75 Vol.-%. Der Inertgasanteil des Gasgemischs G liegt in der Regel bei ≤ 95 Vol.-%, meist bei ≤ 90 Vol.-%.

Erfindungsgemäß geeignete Gasgemische G können somit z.B. aus 3 bis 20 Vol.-% molekularem Sauerstoff, 0 bis 5 Vol.-% Wasserdampf und als Restmenge aus Inertgas bestehen. Bevorzugte Inertgase sind N₂ und CO₂. Insbesondere kommen für das erfindungsgemäße Verfahren alle in der EP-A 169449 sowie in der EP-A 339119 empfohlenen Gasgemische G in Betracht. Ebenfalls können für das erfindungsgemäße Verfahren alle in der EP-A 169449 empfohlenen Regenerierbedingungen angewendet werden.

Die Menge des beim erfindungsgemäßen Verfahren durch das Katalysatorfestbett geführten Gasgemischs G kann 5 oder 100 bis 5000 Nl/l•h, vorzugsweise 20 oder 200 bis 3000 Nl/l•h betragen. Bezugsbasis ist dabei das Volumen des gesamten Katalysatorfestbetts, d.h., einschließlich gegebenenfalls mitverwendeter Abschnitte, die ausschließlich aus Inertmaterial bestehen. Hohe Belastungen mit Gasgemisch G haben sich als vorteilhaft erwiesen.

Als für das erfindungsgemäße Verfahren geeignete Multimetalloxidaktivmassen kommen insbesondere Mo, Bi und Fe enthaltende aktive Multimetalloxide in Betracht. Dies sind vor allem die in der DE-A 10344149 und in der DE-A 10344264 offenbarten Mo, Bi und Fe enthaltenden Multimetalloxidmassen.

Dies sind aber insbesondere auch die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für das erfindungsgemäß zu verwendende Katalysatorfestbett die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1 c aus der EP-A 15565 sowie ein in entsprechender Weise herzustellender Katalysator, dessen Aktivmasse jedoch die Zusammensetzung MO₁₂Ni_{6,5}Zn₂Fe₂Bi₁P_{0,0065}K_{0,06}Ox • 10 SiO₂ aufweist. Ferner sind hervorzuheben das Beispiel mit der laufenden Nr. 3 aus der DE-A 19855913 (Stöchiometrie: Mo₁₂Co₇Fe₃Bi_{0,6}K_{0,08}Si_{1,6}Ox) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 10101695 bzw. WO 02/062737.

Weiterhin sind das Beispiel 1 aus der DE-A 10046957 (Stöchiometrie: [Bi₂W₂O₉ x 2WO₃]_{0,5} • [Mo₁₂Co_{5,6}Fe_{2,94}Si_{1,59}K_{0,08}Oₓ]₁) als Hohlzylinder (Ring) vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 10063162 (Stöchiometrie: Mo₁₂Bi_{1,0}Fe₃Co₇Si_{1,6}K_{0,08}), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, geeignet.

Eine Vielzahl der für die Katalysatoren des für das erfindungsgemäße Verfahren erforderlichen Katalysatorfestbetts geeigneten Multimetalloxidaktivmassen lässt sich unter der allgemeinen Formel I,

Mo₁₂BiₐFe_{b}X¹_{c}X²_{d}X³ₑX⁴_{f}Oₙ (I),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,
a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Üblicherweise werden die Multimetalloxidaktivmassen der allgemeinen Formel I im beim erfindungsgemäßen Verfahren erforderlichen Katalysatorfestbett nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm bzw. 5 mm x 3 mm x 2 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die Katalysatoren des Katalysatorfestbetts des erfindungsgemäßen Verfahrens geeignete Multimetalloxidaktivmassen sind ferner Massen der allgemeinen Formel II,

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} (II),

in der die Variablen folgende Bedeutung haben:
Y¹ = nur Wismut oder Wismut und wenigstens eines der Elemente Tellur, Antimon, Zinn und Kupfer,
Y² = Molybdän oder Molybdän und Wolfram,
Y³ = ein Alkalimetall, Thallium und/oder Samarium,
Y⁴ = ein Erdalkalimetall, Nickel, Kobalt, Kupfer, Mangan, Zink, Zinn, Cadmium und/oder Quecksilber,
Y⁵ = Eisen oder Eisen und wenigstens eines der Elemente Chrom und Cer,
Y⁶ = Phosphor, Arsen, Bor und/oder Antimon,
Y⁷ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in II bestimmt werden und
p,q = Zahlen, deren Verhältnis p/q 0,1 bis 10 beträgt,
   enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte Multimetalloxidmassen II sind solche, in denen Y¹ nur Wismut ist.

Unter diesen werden wiederum jene bevorzugt, die der allgemeinen Formel III,

[Bi_{a"}Z²_{b"}O_{x"}]_{p"}[Z²₁₂Z³_{c"}Z⁴_{d"}Fe_{e"}Z⁵_{f"}Z⁶_{g"}Z⁷_{h"}O_{y"}]_{q"} (III),

in der die Varianten folgende Bedeutung haben:
Z² = Molybdän oder Molybdän und Wolfram,
Z³ = Nickel und/oder Kobalt,
Z⁴ = Thallium, ein Alkalimetall und/oder ein Erdalkalimetall,
Z⁵ = Phosphor, Arsen, Bor, Antimon, Zinn, Cer und/oder Blei,
Z⁶ = Silicium, Aluminium, Titan und/oder Zirkonium,
Z⁷ = Kupfer, Silber und/oder Gold,
a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"=Zahlen, die durch die Wertigkeit und Häufigkeit der von Sauerstoff verschiedenen Element in III bestimmt werden,
p",q"=Zahlen, deren Verhältnis p"/q" 0,1 bis 5, vorzugsweise 0,5 bis 2 beträgt,
entsprechen, wobei diejenigen Massen III ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}) vorliegen, deren Größtdurchmesser im Bereich 1 nm bis 100 µm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913, der DE-A 10344149 und der DE-A 10344264 beschrieben.

Anwendungstechnisch zweckmäßig wird man das erfindungsgemäße Verfahren der heterogen katalysierten Gasphasenpartialoxidation von Propen zu Acrolein in einem mit den Festbettkatalysatoren beschickten Rohrbündelreaktor, wie er z.B. in der EP-A 700 714 bzw. der DE-A 4 431 949 oder der WO 03/057653, oder der WO 03/055835, oder der WO 03/059857, oder der WO 03/076373 beschrieben ist, durchführen.

D.h., in einfachster Weise befindet sich das beim erfindungsgemäßen Verfahren zu verwendende Katalysatorfestbett in den einheitlich beschickten Metallrohren eines Rohrbündelreaktors und um die Metallrohre wird ein Temperiermedium (Einzonenfahrweise), in der Regel eine Salzschmelze, geführt. Salzschmelze (Temperiermedium) und Reaktionsgasgemisch können dabei im einfachen Gleich- oder Gegenstrom geführt werden. Das Temperiermedium (die Salzschmelze) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemischs besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor 0 bis 10°C, häufig 2 bis 8°C, oft 3 bis 6°C beträgt. Die Eintrittstemperatur des Wärmeaustauschmittels in den Rohrbündelreaktor (sie entspricht in dieser Schrift der Temperatur des Katalysatorfestbetts) beträgt in der Regel 250 bis 450°C, häufig 300 bis 400°C bzw. 300 bis 380°C. Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle. Aber auch ionische Flüssigkeiten sind einsetzbar.

Zweckmäßigerweise wird das Reaktionsgasgemisch der Beschickung mit Festbettkatalysator auf die gewünschte Reaktionstemperatur vorerwärmt zugeführt.

Insbesondere im Fall von angestrebten hohen (z.B. ≥ 140 Nl/l • h oder ≥ 160 Nl/l • h, in der Regel jedoch ≤ 600 Nl/l • h) Endbelastungen des Katalysatorfestbetts mit Propen erfolgt die Durchführung des erfindungsgemäßen Verfahrens zweckmäßig in einem Zweizonenrohrbündelreaktor (eine Durchführung im Einzonenrohrbündelreaktor ist jedoch ebenfalls möglich). Eine bevorzugte Variante eines für diesen Zweck erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903582 offenbarten Zweizonenrohrbündelreaktoren sind geeignet.

D.h., in einfacher Weise befindet sich das erfindungsgemäß zu verwendende Katalysatorfestbett in den einheitlich beschickten Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert eine Temperatur- bzw. Reaktionszone.

Vorzugsweise umströmt z.B. ein Salzbad A denjenigen Abschnitt der Rohre (die Reaktionszone A), in welchem sich die oxidative Umsetzung des Propens (beim einfachen Durchgang) bis zum Erreichen eines Umsatzwertes im Bereich von 40 bis 80 mol.% vollzieht und ein Salzbad B umströmt den Abschnitt der Rohre (die Reaktionszone B), in welchem sich die oxidative Anschlußumsetzung des Propens (beim einfachen Durchgang) bis zum Erreichen eines Umsatzwertes von in der Regel wenigstens 90 mol.% vollzieht (bei Bedarf können sich an die Reaktionszonen A, B weitere Reaktionszonen anschließen, die auf individuellen Temperaturen gehalten werden).

Innerhalb der jeweiligen Temperaturzone kann das Salzbad prinzipiell wie bei der Einzonenfahrweise geführt werden. Die Eintrittstemperatur des Salzbades B liegt normalerweise wenigstens 5 bis 10°C oberhalb der Temperatur des Salzbades A. Im übrigen können die Eintrittstemperaturen im für die Einzonenfahrweise empfohlenen Temperaturbereich für die Eintrittstemperatur liegen.

Ansonsten kann die Zwei-Zonen-Hochlastfahrweise z.B. wie in der DE-A 10308836, der EP-A 1106598 oder wie in der WO 01/36364, oder der DE-A 19927624, oder der DE-A 19948523, der DE-A 10313210, der DE-A 10313213 oder wie in der DE-A 19948248 beschrieben durchgeführt werden.

Danach eignet sich das erfindungsgemäße Verfahren für Propenbelastungen des Katalysatorfestbetts von ≥ 70 Nl/l • h, ≥ 90 Nl/l • h, ≥ 110 Nl/l • h, ≥ 130 Nl/l • h, ≥ 140 Nl/l • h, ≥ 160 Nl/l • h, ≥ 180 Nl/l • h, ≥ 240 Nl/l • h, ≥ 300 Nl/l • h, jedoch normalerweise ≤ 600 Nl/l • h. Hier (d.h., generell bei Propenbelastungen in dieser Schrift), anders als sonst in dieser Schrift, ist die Belastung auf das Volumen des Katalysatorfestbetts ausschließlich gegebenenfalls mitverwendeter Abschnitte die ausschließlich aus Inertmaterial bestehen bezogen.

Dabei kann das für das Beschickungsgasgemisch zu verwendende Inertgas zu z.B. ≥ 20 Vol.-%, oder zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Das inerte Verdünnungsgas kann aber auch z.B. zu 2 bis 35 bzw. 20 Gew.-% aus H₂O und zu 65 bis 98 Vol.-% aus N₂ bestehen.

Bei Propenbelastungen des Katalysatorfestbetts oberhalb von 250 Nl/l • h wird für das erfindungsgemäße Verfahren jedoch die Mitverwendung von inerten Verdünnungsgasen wie Propan, Ethan, Methan, Butan, Pentan, CO₂, CO, Wasserdampf und/oder Edelgasen empfohlen. Selbstverständlich können diese Gase aber auch bereits bei geringeren Propenbelastungen mitverwendet werden.

Der Arbeitsdruck kann bei der erfindungsgemäßen Gasphasenpartialoxidation des Propens sowohl unterhalb von Normaldruck (z.B. bis zu 0,5 bar) als auch oberhalb von Normaldruck liegen. Typischerweise wird der Arbeitsdruck bei der Gasphasen-Partialoxidation des Propens bei Werten von 1 bis 5 bar, häufig 1 bis 3 bar liegen.

Normalerweise wird der Reaktionsdruck bei der erfindungsgemäßen Propenpartialoxidation 100 bar nicht überschreiten.

Das molare Verhältnis von O₂:Propen im Reaktionsgasausgangsgemisch, das beim erfindungsgemäßen Verfahren durch das Katalysatorfestbett geführt wird, wird normalerweise ≥ 1 betragen. Üblicherweise wird dieses Verhältnis bei Werten ≤ 3 liegen. Häufig wird das molare Verhältnis von O₂:Propen im vorgenannten Beschickungsgasgemisch erfindungsgemäß 1 bis 2 bzw. 1 bis 1,5 betragen. Vielfach wird man das erfindungsgemäße Verfahren mit einem im Reaktionsgasausgangsgemisch vorliegenden Propen:Sauerstoff:Inertgas (einschließlich Wasserdampf) - Volumenverhältnis (Nl) von 1 :(1 bis 3) : (3 bis 30), vorzugsweise von 1 :(1,5 bis 2,3) : (10 bis 15) ausführen.

Der Propenanteil im Reaktionsgasausgangsgemisch kann z.B. bei Werten von 4 oder 7 bis 20 Vol.-%, häufig bei 5 oder 7 bis 15 Vol.-% bzw. 5 oder 7 bis 12 Vol.-% oder 5 bis 8 Vol.-% liegen (jeweils bezogen auf das Gesamtvolumen).

Eine typische Zusammensetzung des Reaktionsgasausgangsgemischs (unabhängig von der gewählten Belastung) kann z.B. die nachfolgenden Komponenten enthalten:
6 bis 6,5 Vol.-% Propen,
3 bis 3,5 Vol.-% H₂O,
0,3 bis 0,5 Vol.-% CO,
0,8 bis 1,2 Vol.-% CO₂,
0,025 bis 0,04 Vol.-% Acrolein,
10,4 bis 10,7 Vol.-% O₂ und
als Restmenge ad 100% molekularer Stickstoff,
oder: 5,4 Vol.-% Propen,
10,5 Vol.-% Sauerstoff,
1,2 Vol.-% COₓ,
81,3 Vol.-% N₂ und
1,6 Vol.-% H₂O.

Das Reaktionsgasausgangsgemisch kann aber auch wie folgt zusammengesetzt sein:
6 bis 15 Vol.-% Propen,
4 bis 30 Vol.-% (häufig 6 bis 15 Vol.-%) Wasser, ≥ 0 bis 10 Vol.-% (vorzugsweise ≥ 0 bis 5 Vol.-%) an von Propen, Wasser, Sauerstoff und Stickstoff verschiedenen Bestandteilen, soviel molekularem Sauerstoff, dass das molare Verhältnis von enthaltenem molekularem Sauerstoff zu enthaltenem molekularem Propen 1,5 bis 2,5 beträgt und als Restmenge bis zur 100 Vol.-% Gesamtmenge aus molekularem Stickstoff.

Eine andere mögliche Reaktionsgasausgangsgemischzusammensetzung kann enthalten:
6,0 Vol.-% Propen,
60 Vol.-% Luft und
34 Vol.-% H₂O.

Alternativ können auch Reaktionsgasausgangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 verwendet werden.

Weitere erfindungsgemäß geeignete Reaktionsgasausgangsgemische können im nachfolgenden Zusammensetzungsraster liegen:
7 bis 11 Vol.-% Propen,
6 bis 12 Vol.-% Wasser,
≥ 0 bis 5 Vol.-% an von Propen, Wasser, Sauerstoff und Stickstoff verschiedenen Bestandteilen,
   soviel molekularem Sauerstoff, dass das molare Verhältnis von enthaltenem Sauerstoff zu enthaltenem molekularem Propen 1,4 bis 2,2 beträgt, und
   als Restmenge bis zur 100 Vol.-% Gesamtmenge molekularer Stickstoff.

Als im Reaktionsgasausgangsgemisch zu verwendendes Propen kommen vor allem polymer grade Propen und chemical grade Propen in Betracht, wie es z.B. die DE-A 10232748 beschreibt.

Als Sauerstoffquelle wird normalerweise Luft eingesetzt.

Zur Bereitung des Katalysatorfestbetts können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden (aus Inertmaterial bestehenden), Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

In der Regel ist es günstig, wenn sich die chemische Zusammensetzung der verwendeten Aktivmasse über das Katalysatorfestbett nicht verändert. D.h., die für einen einzelnen Katalysatorformkörper verwendete Aktivmasse kann zwar ein Gemisch aus verschiedenen, die Elemente Mo und/oder W sowie wenigstens eines der Elemente Bi, Fe, Sb, Sn und Cu enthaltenden, Multimetalloxiden sein, für alle Katalysatorformkörper des Katalysatorfestbetts ist dann jedoch vorteilhaft das gleiche Gemisch zu verwenden.

Bevorzugt nimmt normalerweise die volumenspezifische (d.h., die auf die Einheit des Volumens normierte) Aktivität innerhalb des Katalysatorfestbetts in Strömungsrichtung des Reaktionsgasausgangsgemischs kontinuierlich, abrupt oder stufenförmig zu.

Die volumenspezifische Aktivität kann dabei z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen des Festbetts enthaltene Aktivmasse bzw. Katalysatoraktivität.

Eine in Strömungsrichtung des Reaktionsgasgemischs über des Katalysatorfestbett wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Eine Zunahme der volumenspezifischen Aktivität ist aber auch z.B. dadurch möglich, dass man bei gleich bleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für das Katalysatorfestbett aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum mit inerten Verdünnungskörpern verdünnt werden.

Vorab und/oder im Anschluss an die Aktivmasse aufweisenden Abschnitte des Katalysatorfestbetts können sich ausschließlich aus Inertmaterial (z.B. nur Verdünnungsformkörpern) bestehende Schüttungen befinden (sie werden in dieser Schrift begrifflich, soweit nichts anderes gesagt wird, dem Katalysatorfestbett zugerechnet). Diese können ebenfalls auf die Temperatur des Katalysatorfestbetts gebracht sein. Dabei können die für die Inertschüttung verwendeten Verdünnungsformkörper die gleiche Geometrie wie die zu den Aktivmasse aufweisenden Abschnitte des Katalysatorfestbetts verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Vielfach ist beim erfindungsgemäßen Verfahren der Aktivmasse aufweisende Abschnitt des Katalysatorfestbetts in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge des Aktivmasse aufweisenden Abschnitts der Festbettkatalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, vorzugsweise 10 bis 40 Gew.-% oder 20 bis 40 Gew.-% und besonders bevorzugt 25 bis 35 Gew.-% beträgt. Im Anschluss an diese erste Zone befindet sich dann häufig vorteilhaft bis zum Ende der Länge des Aktivmasse aufweisenden Abschnitts des Katalysatorfestbetts (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn im Katalysatorfestbett als Katalysatorformkörper Vollkatalysatorringe oder Schalenkatalysatorringe eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-%-Punkte tiefer liegt, als der Aktivmassenanteil der Schalenkatalysatorformkörper am Ende des Katalysatorfestbetts.

Eine reine Inertmaterialschüttung, deren Länge, bezogen auf die Gesamtlänge des Katalysatorfestbetts, zweckmäßig 1 oder 5 bis 20 % beträgt, leitet in Strömungsrichtung des Reaktionsgasgemischs in der Regel das Katalysatorfestbett ein. Sie wird normalerweise als Aufheizzone für das Reaktionsgasgemisch genutzt.

Üblicherweise sind die Kontaktrohre in den Rohrbündelreaktoren aus ferritischem Stahl gefertigt und weisen in typischer Weise eine Wanddicke von 1 bis 3 mm auf. Ihr Innendurchmesser beträgt in der Regel (einheitlich) 20 bis 30 mm, häufig 21 bis 26 mm. Anwendungstechnisch zweckmäßig beläuft sich die im Rohrbündelbehälter untergebrachte Anzahl an Kontaktrohen auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Die Belastung des Katalysatorfestbetts (hier ausschließlich reiner Inertabschnitte) mit Reaktionsgasgemisch wird beim erfindungsgemäßen Verfahren in typischer Weise 1000 bis 10000 Nl/l•h, meist 1000 bis 5000 Nl/l•h, häufig 1500 bis 4000 Nl/l•h betragen.

Bei der Ausübung des erfindungsgemäßen Verfahrens wird man ein frisches Katalysatorfestbett nach seiner Formierung normalerweise so betreiben, dass man nach Festlegung der Zusammensetzung des Reaktionsgasausgangsgemischs und Festlegung der Belastung des Katalysatorfestbetts mit Reaktionsgasausgangsgemisch die Temperatur des Katalysatorfestbetts (bzw. die Eintrittstemperatur des Temperiermediums in die Temperierzone des Rohrbündelreaktors) so einstellt, dass der Umsatz U^{Pro} des Propens bei einmaligem Durchgang des Reaktionsgasgemischs durch das Katalysatorfestbett wenigstens 90 mol.-% beträgt. Bei Verwendung günstiger Katalysatoren sind auch Werte für U^{Pro} ≥ 92 mol.-%, oder ≥ 93 mol.-%, oder ≥ 94 mol.-%, oder ≥ 96 mol.-% und häufig sogar mehr möglich.

Bei kontinuierlicher Ausübung der heterogen katalysierten Partialoxidation von Propen zu Acrolein sowie gegebenenfalls Acrylsäure wird man die Zusammensetzung des Reaktionsgasausgangsgemischs und die Belastung des Katalysatorfestbetts mit Reaktionsgasausgangsgemisch im wesentlichen konstant beibehalten (gegebenenfalls wird die Belastung an die fluktuierende Marktnachfrage angepasst). Einem Abfallen der Aktivität des Katalysatorfestbetts über die Zeit wird man unter diesen Produktionsbedingungen normalerweise dadurch entgegenwirken, dass man von Zeit zu Zeit die Temperatur des Katalysatorfestbetts (die Eintrittstemperatur des Temperiermediums in die Temperaturzone des Rohrbündelreaktors) erhöht (die Fließgeschwindigkeit des Temperiermediums wird normalerweise im wesentlichen ebenfalls beibehalten), um den Propenumsatz bei einmaligem Durchgang des Reaktionsgasgemischs im angestrebten Zielkorridor (d. h., bei U^{Pro} ≥ 90 mol-%, bzw. ≥ 92 mol-%, bzw. ≥ 93 mol-%, bzw. ≥ 94 mol-%, bzw. ≥ 96 mol-%) zu halten. Eine solche Vorgehensweise zieht jedoch die Eingangs dieser Schrift beschriebenen Nachteile mit sich.

Erfindungsgemäß vorteilhaft wird man deshalb so verfahren, dass man wenigstens einmal pro Kalenderquartal die Gasphasenpartialoxidation unterbricht, um bei einer Temperatur des Katalysatorfestbetts von 250 bis 550 °C ein aus molekularem Sauerstoff, Inertgas und gegebenenfalls Wasserdampf bestehendes Gasgemisch G durch das Katalysatorfestbett zu führen. Anschließend wird die Partialoxidation unter weitgehendem Beibehalt der Verfahrensbedingungen (vorzugsweise wird die Propenbelastung langsam auf ihren Wert wieder eingestellt, z. B. so wie in der DE-A 10337788 beschrieben) fortgesetzt und die Temperatur des Katalysatorfestbetts so eingestellt, dass der Propenumsatz den angestrebten Zielwert erreicht. In der Regel wird dieser Temperaturwert bei gleichem Umsatz bei einem etwas niedrigeren Wert liegen als die Temperatur, die das Katalysatorfestbett vor der Unterbrechung der Partialoxidation und der erfindungsgemäßen Behandlung mit dem Gasgemisch G aufwies. Von diesem Temperaturwert des Katalysatorfestbetts ausgehend wird die Partialoxidation unter weitgehendem Beibehalt der übrigen Bedingungen fortgesetzt und dabei dem Abfall der Aktivität des Katalysatorfestbetts über die Zeit in zweckmäßiger Weise wiederum dadurch entgegengewirkt, dass man von Zeit zu Zeit die Temperatur des Katalysatorfestbetts erhöht. Innerhalb eines Kalenderquartals wird die Partialoxidation erfindungsgemäß wiederum wenigstens einmal unterbrochen, um das Gasgemisch G in erfindungsgemäßer Weise durch das Katalysatorfestbett zu führen. Danach wird die Partialoxidation erfindungsgemäß vorteilhaft wie beschrieben wieder aufgenommen u. s. w.

Es überrascht, dass beim erfindungsgemäßen Verfahren die Ausprägung der Heißpunktausdehnung im Langzeitbetrieb einer heterogen katalysierten Gasphasenpartialoxidation von Propen zu Acrolein und gegebenenfalls Acrylsäure ein günstigeres Verhalten zeigt, als bei den Verfahren gemäß des Standes der Technik. Das erfindungsgemäße Verfahren ermöglicht so einerseits längere Standzeiten eines Katalysatorfestbetts in einem Reaktor, bevor dieses teilweise oder vollständig ausgetauscht werden muss. Auf der anderen Seite ist auch der über die Zeit integral erzielte Propenumsatz ein erhöhter und die Selektivität der Zielproduktbildung wird ebenfalls gefördert. Dazu trägt unter anderem bei, dass der Ort des Heißpunktes beim erfindungsgemäßen Verfahren über die Zeit stationär bleibt oder normalerweise in Richtung der Eintrittsstelle des Reaktionsgasgemischs in das Katalysatorfestbett wandert. Damit wandert der Heißpunkt im Reaktionsgasgemisch zunehmend in den Bereich vor, in dem der Acroleingehalt noch wenig ausgeprägt ist. Dies mindert die Möglichkeit, dass bereits gebildete Acrolein unter der Einwirkung der Heißpunkttemperatur partiell unerwünschte Vollverbrennung erleidet. Die Ermittlung der Heißpunkttemperatur kann beim erfindungsgemäßen Verfahren in Rohrbündelreaktoren z.B. mittels Thermorohren erfolgen, wie sie in der EP-A 873 783, der WO 03-076373 und in der EP-A 1 270 065 beschrieben sind. Die Anzahl solcher Thermorohre innerhalb eines Rohrbündelreaktors beträgt zweckmäßig 4 bis 20. Mit Vorteil befinden sie sich im Rohrbündelinneren gleichmäßig verteilt angeordnet.

Häufig wird man beim erfindungsgemäßen Verfahren die Erhöhung der Katalysatorfestbetttemperatur so durchführen, dass der Propenumsatz bei einmaligem Durchgang des Reaktionsgasgemischs durch das Katalysatorbett 90 mol-%, bzw. 92 mol-%, bzw. 93 mol-%, bzw. 94 mol-%, bzw. 96 mol-% oder 97 mol-% nicht unterschreitet. D.h., normalerweise wird man die Katalysatorfestbetttemperatur wenigstens einmal erhöhen bevor 7500 oder bevor 7000, meist bevor 6000 und vielfach bevor 5000 oder 4000 Betriebsstunden der Partialoxidation erreicht sind.

Abschließend sei festgehalten, dass die Erhöhung der Katalysatorfestbetttemperatur über die Zeit beim erfindungsgemäßen Verfahren unter Anwendung besonders günstiger Katalysatoren (z.B. den in dieser Schrift empfohlenen) bevorzugt (meist im wesentlichen kontinuierlich und) so durchgeführt wird, dass der Propengehalt im Produktgasgemisch den Wert 10000 Gew.-ppm, vorzugsweise 6000 Gew.-ppm und besonders bevorzugt 4000 bzw. 2000 Gew.-ppm nicht übersteigt. Ferner sollte der Restsauerstoff im Produktgasgemisch in der Regel wenigstens 1 Vol.-%, vorzugsweise wenigstens 2 Vol.-% und besonders bevorzugt wenigstens 3 Vol.-% betragen.

Das erfindungsgemäße Verfahren ist insbesondere dann vorteilhaft, wenn es mit einer Belastung des Katalysatorfestbetts mit Propen von ≥ 120 N/l•h, bzw. ≥ 130 Nl/l•h, bzw. ≥ 140 Nl/l•h betrieben wird. Abschließend sei festgehalten, dass das erfindungsgemäße Verfahren in entsprechender Weise zum Langzeitbetrieb einer heterogen katalysierten Gasphasenpartialoxidation von iso-Buten, dem Methylether des tert.Butanols und/oder von tert.Butanol zu Methacrolein geeignet ist (insbesondere bei Anwendung derselben Katalysatorsysteme; es kommen aber auch die Verfahrensbedingungen und Katalysatorsysteme der WO 03/039744 in Betracht). Generell wird man das frisch beschickte Katalysatorbett so gestalten, dass, wie in der EP-A 990636 und EP-A 1106598 beschrieben, sowohl die Heißpunktausbildung als auch deren Temperatursensitivität möglichst gering sind. Ferner wird man sowohl bei der Erstinbetriebnahme als auch bei der Wiederinbetriebnahme nach Ausübung des erfindungsgemäßen Verfahrens die Belastung des Katalysatorfestbetts mit Propen vorteilhaft bei Werten ≤ 100 Nl/l•h belassen, bis sich ein stabiler Betrieb eingestellt hat.

### Beispiele

### A) Herstellung des verwendeten Katalysators

Herstellung eines ringförmigen Vollkatalysators mit der nachfolgenden Stöchiometrie des aktiven Multimetalloxids II:

[Bi₂W₂O₉ • 2WO₃]_{0,5}[Mo₁₂Co_{5,5}Fe_{2,94}Si_{1,59}K_{0,06}Oₓ]₁.

### 1. Herstellung einer Ausgangsmasse 1

In 775 kg einer wäßrigen salpetersauren Wismutnitratlösung (11,2 Gew.-% Bi, freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gegenstrom bei einer Gaseintrittstemperatur von 300 ± 10°C und einer Gasaustrittstemperatur von 100 ± 10°C. Das erhaltene Sprühpulver (Partikelgröße im wesentlichen einheitlich 30 µm), das einen Glühverlust von 12 Gew.-% aufwies (3 h bei 600°C unter Luft glühen), wurde anschließend mit 16,8 Gew.-% (bezogen auf das Pulver) Wasser in einem Kneter angeteigt und mittels eines Extruders (Drehmoment: ≤ 50 Nm) zu Strängen des Durchmessers 6 mm extrudiert. Diese wurden in Abschnitte von 6 cm geschnitten, auf einen 3-zonigen Bandtrockner bei einer Verweilzeit von 120 min bei Temperaturen von 90-95°C (Zone 1) und 125°C (Zone 2) und 125°C (Zone 3) an Luft getrocknet und dann bei einer Temperatur im Bereich von 780 bis 810°C thermisch behandelt (calciniert; im luftdurchströmten Drehrohrofen (1,54 m³ Innenvolumen, 200 Nm³ Luft/h)). Wesentlich bei der genauen Einstellung der Calcinationstemperatur ist, daß sie an der angestrebten Phasenzusammensetzung des Calcinationsprodukts orientiert zu erfolgen hat. Gewünscht sind die Phasen WO₃ (monoklin) und Bi₂W₂O₉, unerwünscht ist das Vorhandensein von γ-Bi₂WO₆ (Russellit). Sollte daher nach der Calcination die Verbindung γ-Bi₂WO₆ anhand eines Reflexes im Pulverröntgendiffraktogramm bei einem Reflexwinkel von 2Θ = 28,4° (CuKa-Strahlung) noch nachweisbar sein, so ist die Präparation zu wiederholen und die Calcinationstemperatur innerhalb des angegebenen Temperaturbereichs oder die Verweilzeit bei gleichbleibender Calcinationstemperatur zu erhöhen, bis das Verschwinden des Reflexes erreicht wird. Das so erhaltene vorgebildete calcinierte Mischoxid wurde gemahlen, so daß der X₅₀-Wert (vgl. Ullmann's Encyclopedia of Industrial Chemistry, 6th Edition (1998) Electronic Release, Kapitel 3.1.4 oder DIN 66141) der resultierenden Körnung 5 mm betrug. Das Mahlgut wurde dann mit 1 Gew.-% (bezogen auf das Mahlgut) feinteiligem SiO₂ der Fa. Degussa vom Typ Sipernat^{®} (Rüttelgewicht 150 g/l; X₅₀-Wert der SiO₂-Partikel betrug 10 µm, die BET-Oberfläche betrug 100 m²/g) vermischt.

### 2. Herstellung einer Ausgangsmasse 2

Eine Lösung A wurde hergestellt, indem man bei 60°C unter Rühren in 600 l Wasser 213 kg Ammoniumheptamolybdattetrahydrat (81,5 Gew.-% MoO₃) löste und die resultierende Lösung unter Aufrechterhaltung der 60°C und Rühren mit 0,97 kg einer 20°C aufweisenden wässrigen Kaliumhydroxidlösung (46,8 Gew.-% KOH) versetzte.

Eine Lösung B wurde hergestellt indem man bei 60°C in 262,9 kg einer wässrigen Co-(II)-baltnitratlösung (12,4 Gew.-% Co) 116,25 kg einer wäßrigen Eisen-(III)-nitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wäßrigen Gemisch 19,16 kg eines Kieselgels der Fa. Dupont vom Typ Ludox (46,80 Gew.-% SiO₂, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintrittstemperatur: 400 ± 10°C, Gasaustrittstemperatur: 140 ± 5°C). Das resultierende Sprühpulver wies einen Glühverlust von ca. 30 Gew.-% (3 h bei 600°C unter Luft glühen) und eine im wesentlichen einheitliche Körnung von 30 µm auf.

### 3. Herstellung der Multimetalloxidaktivmasse II

Die Ausgangsmasse 1 wurde mit der Ausgangsmasse 2 in den für eine Multimetalloxidaktivmasse der Stöchiometrie

[Bi₂W₂O₉ · 2WO₃]_{0,5}[Mo₁₂Co_{5,5}Fe_{2,94}Si_{1,59}K_{0,08}Oₓ]₁

erforderlichen Mengen in einem Mischer mit Messerköpfen homogen vermischt. Bezogen auf die vorgenannte Gesamtmasse wurden zusätzlich 1 Gew.-% feinteiliges Graphit der Fa. Timcal AG (San Antonio, US), vom Typ TIMREX P44 (Siebanalyse: min. 50 Gew.-% < 24 mm, max. 10 Gew.-% ≥ 24 µm und ≤ 48 µm, max. 5 Gew.-% > 48 µm, BET-Oberfläche: 6 bis 13 m²/g) homogen eingemischt. Das resultierende Gemisch wurden dann in einem Kompaktor (Fa. Hosokawa Bepex GmbH, D-74211 Leingarten) vom Typ Kompaktor K200/100 mit konkaven, geriffelten Glattwalzen gefahren (Spaltweite: 2,8 mm, Siebweite: 1,0 mm, Siebweite Unterkorn: 400 µm, Presssollkraft: 60 kN, Schneckendrehzahl: 65 bis 70 Umdrehungen je Minute). Das resultierende Kompaktat wies eine Härte von 10 N und eine im wesentlichen einheitliche Körnung von 400 µm bis 1 mm auf.

Das Kompaktat wurde anschließend mit, bezogen auf sein Gewicht, weiteren 2 Gew.-% desselben Graphit vermischt und anschließend in einem Kilian Rundläufer (Tablettiermaschine) vom Typ R x 73, der Fa. Kilian, D-50735 Köln, unter Stickstoffatmosphäre zu ringförmigen Vollkatalysatorvorläuferformkörpern der Geometrie (Außendurchmesser x Länge x Innendurchmesser) 5 mm x 3 mm x 2 mm mit einer Seitendruckfestigkeit von 19 N ± 3 N verdichtet.

Als Seitendruckfestigkeit wird dabei in dieser Schrift die Druckfestigkeit bei Stauchung des ringförmigen Vollkatalysatorvorläuferformkörpers senkrecht zur Zylinderfläche (d.h., parallel zur Fläche der Ringöffnung) verstanden.

Dabei beziehen sich alle Seitendruckfestigkeiten dieser Schrift auf eine Bestimmung mittels einer Material-Prüfmaschine der Firma Zwick GmbH & Co. (D-89079 UIm) des Typs Z 2.5 / TS1S. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wurde dabei entsprechend der DIN EN ISO 7500-1 kalibriert und war für den Messbereich 1-500N einsetzbar (relative Messunsicherheit: ± 0,2 %).

Die Messungen wurden mit folgenden Parametern durchgeführt:
Vorkraft: 0,5 N.
Vorkraft-Geschwindigkeit: 10 mm/min.
Prüfgeschwindigkeit: 1,6 mm/min.

Dabei wurde der obere Stempel zunächst langsam bis kurz vor Zylinderfläche des ringförmigen Vollkatalysatorvorläuferformkörpers abgesenkt. Dann wurde der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der Vollkatalysatorvorläuferformkörper Rissbildung zeigt, ist die Seitendruckfestigkeit (SDF).

Zur abschließenden thermischen Behandlung wurden jeweils 1000 g der Vollkatalysatorvorläuferformkörper in einem Luft durchströmten Muffelofen (60 l Innenvolumen, 1 l/h Luft pro Gramm Vollkatalysatorvorläuferformkörper) zunächst mit einer Aufheizrate von 180°C/h von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurden. Diese Temperatur wurde ebenfalls 1 h aufrechterhalten, bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten.

Dabei wurden aus den ringförmigen Vollkatalysatorvorläuferformkörpern ringförmige Vollkatalysatoren erhalten.

Die spezifische Oberfläche O, das Porengesamtvolumen V, der Porendurchmesser d^{max} der den größten Beitrag zum Porengesamtvolumen leistet, sowie die prozentualen Anteile derjenigen Porendurchmesser am Porengesamtvolumen, deren Durchmesser > 0,1 und ≤ 1 µm betragen, lautete für die erhaltenen ringförmigen Vollkatalysatoren wie folgt:
O = 7,6 cm²/g.
V = 0,27 cm³/g.
d^{max} [µm] = 0,6.
V^{0,1}₁-% = 79.

Außerdem betrug das Verhältnis R von scheinbarer Massendichte zu wahrer Massendichte p (so wie es in der EP-A 1340538 definiert ist) 0,66.

In großtechnischer Menge wurde derselbe ringförmige Katalysator durch thermische Behandlung wie in Beispiel 1 der DE-A 10046957 (die Schütthöhe in der Zersetzung (Kammern 1 bis 4) belief sich dabei jedoch vorteilhaft auf 44 mm bei einer Verweilzeit pro Kammer von 1,46 h und in der Calcination (Kammern 5 bis 8) belief sie sich vorteilhaft auf 130 mm bei einer Verweilzeit von 4,67 h) beschrieben mittels einer Bandcalziniervorrichtung hergestellt; die Kammern besaßen eine Grundfläche (bei einer einheitlichen Kammerlänge von 1,40 m) von 1,29 m² (Zersetzung) und 1,40 m² (Calcination) und wurden von unten durch das grobmaschige Band von 75 Nm³/h Zuluft durchströmt, die mittels rotierender Ventilatoren angesaugt wurden. Innerhalb der Kammern war die zeitliche und örtliche Abweichung der Temperatur vom Sollwert stets ≤ 2°C. Im übrigen wurde wie in Beispiel 1 der DE-A 10046957 beschrieben verfahren.

### B) Durchführung der Partialoxidation

### I. Beschreibung der allgemeinen Verfahrensbedingungen

| | |
|---|---|
| Verwendetes Wärmeaustauschmittel: | Salzschmelze, bestehend aus 60 Gew.-% Kaliumnitrat und 40 Gew.-% Natriumnitrit. |
| Material der Kontaktrohre: | ferritischer Stahl. |
| Abmessungen der Kontaktrohre: | 3200 mm Länge; |
| | 25 mm Innendurchmesser; |
| | 30 mm Außendurchmesser (Wandstärke: 2,5 mm). |
| | |
| Anzahl der Kontaktrohre im Rohrbündel: | 25500. |
| Reaktor: | Zylinderförmiger Behälter eines Durchmessers von 6800 mm; ringförmig angeordnetes Rohrbündel mit einem freien zentralen Raum. |
| | |
| | Durchmesser des zentralen freien Raumes: 1000 mm. Abstand der am weitesten außen liegenden Kontaktrohre zur Behälterwand: 150 mm. Homogene Kontaktrohrverteilung im Rohrbündel (6 äquidistante Nachbarrohre pro Kontaktrohr). |
| | |
| | Kontaktrohrteilung: 38 mm. |
| | |
| | Die Kontaktrohre waren mit ihren Enden in Kontaktrohrböden der Dicke 125 mm abdichtend befestigt und mündeten mit ihren Öffnungen in je eine am oberen bzw. unteren Ende mit dem Behälter verbundenen Haube. |
| | |
| | Zuführung des Wärmeaustauschmittels zum Rohrbündel: |
| | |
| | Das Rohrbündel war durch drei zwischen den Kontaktrohrböden längs derselben aufeinanderfolgend angebrachte Umlenkscheiben (Dicke jeweils 10 mm) in 4 äquidistante (jeweils 730 mm) Längsabschnitte (Zonen) geteilt. |

Die unterste und die oberste Umlenkscheibe wies Ringgeometrie auf, wobei der Ringinnendurchmesser 1000 mm betrug und der Ringaußendurchmesser sich bis zur Behälterwand abdichtend erstreckte. Die Kontaktrohre waren an den Umlenkscheiben nicht abdichtend befestigt. Vielmehr waren eine Spaltbreite < 0,5 mm aufweisende Spalte so belassen, daß die Querströmungsgeschwindigkeit der Salzschmelze innerhalb einer Zone möglichst konstant war.

Die mittlere Umlenkscheibe war kreisförmig und erstreckte sich bis zu den am weitesten außen liegenden Kontaktrohren des Rohrbündels.

Die Kreisführung der Salzschmelze wurde durch zwei Salzpumpen bewerkstelligt, von denen jede eine Rohrbündellängshälfte versorgte.

Die Pumpen drückten die Salzschmelze in einen um den Reaktormantel unten angebrachten Ringkanal, der die Salzschmelze über den Behälterumfang verteilte. Durch im Reaktormantel befindliche Fenster gelangte die Salzschmelze im untersten Längsabschnitt zum Rohrbündel. Die Salzschmelze floß dann der Vorgabe der Umlenkbleche folgend in der Abfolge
- von außen nach innen,
- von innen nach außen,
- von außen nach innen,
- von innen nach außen,
im wesentlichen mäanderförmig, über den Behälter betrachtet, von unten nach oben. Durch im obersten Längsabschnitt um den Behälterumfang angebrachte Fenster sammelte sich die Salzschmelze in einem oberen um den Reaktormantel angebrachten Ringkanal und wurde nach Abkühlung auf die ursprüngliche Eintrittstemperatur durch die Pumpen wieder in den unteren Ringkanal gedrückt.

Die Zusammensetzung des Reaktionsgasausgangsgemisches (Gemisch aus Luft, chemical grade Propylen und Kreisgas) lag über die Betriebszeit in folgendem Raster:
5 bis 7 Vol.-% Propen (chemical grade),
10 bis 14 Vol.-% Sauerstoff,
1 bis 2 Vol.-% COₓ,
1 bis 3 Vol.-% H₂O, und
wenigstens 80 Vol.-% N₂.

| | |
|---|---|
| Reaktorbeschickung: | Salzschmelze und Reaktionsgasgemisch wurden über den Reaktor betrachtet im Gegenstrom geführt. Die Salzschmelze trat unten ein, das Reaktionsgasgemisch oben. |
| | Die Eintrittstemperatur der Salzschmelze betrug am Anfang (nach beendeter Formierung des Katalysatorfestbetts) 337°C. |
| | Die zugehörige Austrittstemperatur der Salzschmelze lag am Anfang bei 339°C. |
| | Die Pumpleistung betrug 6200 m³ Salzschmelze/h. Das Reaktionsgasausgangsgemisch wurde dem Reaktor mit einer Temperatur von 300°C zugeführt. |
| | |
| Propenlast des Katalysatorfestbetts: | 90 bis 120 Nl/l•h |

| | |
|---|---|
| Kontaktrohrbeschickung mit Katalysatorfestbett (von oben nach unten): | Zone A: 50 cm |
| | Vorschüttung aus Steatit-Ringen der Geometrie 7 mm x 7 mm x 4 mm (Außendurchmesser x Länge x Innendurchmesser) |
| | |
| | Zone B: 100 cm |
| | Katalysatorbeschickung mit einem homogenen Gemisch aus 30 Gew.-% an Steatit-Ringen der Geometrie 5 mm x 3 mm x 2 mm (Außendurchmesser x Länge x Innendurchmesser) und 70 Gew.-% des hergestellten ringförmigen Vollkatalysator). |
| | |
| | Zone C: 170 cm |
| | Katalysatorbeschickung mit dem hergestellten ringförmigen (5 mm x 3 mm x 2 mm = Außendurchmesser x Länge x Innendurchmesser) Vollkatalysator. |

Die Thermorohre (ihre Anzahl belief sich auf 10, die im zentralen Bereich des Rohrbündels gleichmäßig verteilt waren) waren wie folgt gestaltet und beschickt:
(mit ihnen wurde die Heißpunkttemperatur ermittelt; sie ist ein arithmetischer Mittelwert aus voneinander unabhängigen Messungen in den 10 Thermorohren)
   Jedes der 10 Thermorohre wies eine zentrale Thermohülle mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte).
   Wenigstens 13 und höchstens 30 der jeweils 40 Temperaturmessstellen befand sich im Bereich des ersten Meters des aktiven Abschnitts des Katalysatorfestbetts (in Strömungsrichtung des Reaktionsgasgemischs).
   Der Innendurchmesser eines Thermorohres betrug 27 mm. Die Wanddicke und das Rohrmaterial war wie bei den Arbeitsrohren beschaffen.
   Der Außendurchmesser der Thermohülse betrug 4 mm.
   Ein Thermorohr wurde mit dem hergestellten ringförmigen Vollkatalysator befüllt. Zusätzlich wurde in das Thermorohr aus dem ringförmigen Vollkatalysator erzeugter Katalysatorsplit der Längstausdehnung 0,5 bis 5 mm beigefüllt.
   Die Beifüllung des Katalysatorsplits erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu 5 bis 20 Gew.-% an Katalysatorsplit erforderlich). Gleichzeitig war die jeweilige Gesamtfüllhöhe von Aktiv- und Inertabschnitten in den Arbeits- und Thermorohren gleich bemessen und das Verhältnis von im Rohr enthaltener Gesamtmenge an Aktivmasse zu Wärmeübergangsfläche des Rohres bei Arbeits- und Thermorohren auf den im wesentlichen selben Wert eingestellt.

### II. Langzeitbetrieb (Ergebnisse)

Das Umsatzziel für das bei einmaligem Durchgang des Reaktionsgasgemischs durch das Katalysatorfestbett umzusetzende Propen wurde auf 97,5 mol-% festgelegt.

Durch sukzessives Erhöhen der Eintrittstemperatur der Salzschmelze konnte dieser Umsatzwert bei kontinuierlicher Durchführung des Verfahrens über die Zeit aufrechterhalten werden. Einmal je Kalendermonat wurde die Partialoxidation unterbrochen, die zuletzt angewandte Eintrittstemperatur der Salzschmelze beibehalten und für eine Zeitdauer t_{G} von 24 h bis 48 h ein Gasgemisch G aus 6 Vol.-% O₂ und 95 Vol.-% N₂ mit einer Belastung des Katalysatorfestbetts von 30 Nl/l•h durch das Katalysatorfestbett geführt.

Die Eintrittstemperatur der Salzschmelze und die Heißpunkttemperatur sowie die Selektivität S^{AC+AA} der Summe aus Acroleinbildung und Acrylsäurenebenproduktbildung entwickelten sich dabei wie folgt (die Temperaturangaben (abgesehen für den Anfang) beziehen sich in allen Fällen jeweils auf den Zeitpunkt, der unmittelbar vor der Unterbrechung der Partialoxidation und der Behandlung des Katalysatorfestbetts mit dem Gasgemisch G lag):

| | |
|---|---|
| Anfang: | Salzschmelzeeintrittstemperatur = 318°C |
| | Heißpunkttemperatur = 368°C |
| | S^{AC+AA} = 94,6 mol-% |
| nach 1 Jahr Betriebsdauer : | Salzschmelzeeintrittstemperatur = 322°C |
| | Heißpunkttemperatur = 371 °C |
| | S^{AC+AA} = 94,8 mol-% |
| | |
| nach 2 Jahren Betriebsdauer : | Salzschmelzeeintrittstemperatur = 326°C |
| | Heißpunkttemperatur = 373°C |
| | S^{AC+AA} = 95,0 mol-% |
| | |
| nach 3 Jahren Betriebsdauer: | Salzschmelzeeintrittstemperatur = 332°C |
| | Heißpunkttemperatur = 377°C |
| | S^{AC+AA} = 95, 4 mol-% |
| | |
| nach 4 Jahren Betriebsdauer : | Salzschmelzeeintrittstemperatur = 339°C |
| | Heißpunkttemperatur = 379°C |
| | S^{AC+AA} = 95,6 mol-% |
| | |
| nach 5 Jahren Betriebsdauer : | Salzschmelzeeintrittstemperatur = 345°C |
| | Heißpunkttemperatur = 385°C |
| | S^{AC+AA} =95,6 mol-% |

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Ein Verfahren zum Langzeitbetrieb einer heterogen katalysierten Gasphasenpartialoxidation von Propen zu Acrolein, bei dem man ein Propen, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Molybdän und/oder Wolfram sowie wenigstens eines der Elemente Wismut, Tellur, Antimon, Zinn und Kupfer enthält, und bei dem man, um der Deaktivierung des Katalysatorfestbetts entgegenzuwirken, die Temperatur des Katalysatorfestbetts über die Zeit erhöht, **dadurch gekennzeichnet, dass** man die Gasphasenpartialoxidation wenigstens einmal pro Kalenderquartal unterbricht und bei einer Temperatur des Katalysatorfestbetts von 250 bis 550°C ein aus molekularem Sauerstoff, Inertgas und gegebenenfalls Wasserdampf bestehendes Gasgemisch G durch das Katalysatorfestbett führt.

2. Verfahren nach Anspruch 1, **dadurch gekennzeichnet, dass** man die Gasphasenpartialoxidation wenigstens einmal pro Kalendermonat unterbricht und bei einer Temperatur des Katalysatorfestbetts von 250 bis 550°C ein aus molekularem Sauerstoff, Inertgas und gegebenenfalls Wasserdampf bestehendes Gasgemisch G durch das Katalysatorfestbett führt.

3. Verfahren nach Anspruch 1 oder 2, **dadurch gekennzeichnet, dass** die Zeitdauer, während der das Gasgemisch G durch das Katalysatorfestbett geführt wird, 2 h bis 120 h beträgt.

4. Verfahren nach einem der Ansprüche 1 bis 3, **dadurch gekennzeichnet, dass** das Gasgemisch G, das durch das Katalysatorfestbett geführt wird, wenigstens 4 Vol.-% Sauerstoff enthält.

5. Verfahren nach einem der Ansprüche 1 bis 4, **dadurch gekennzeichnet, dass** es in einem Rohrbündelreaktor durchgeführt wird.

6. Verfahren nach einem der Ansprüche 1 bis 5, **dadurch gekennzeichnet, dass** die Aktivmasse der Katalysatoren ein Mo, Bi und Fe enthaltendes Multimetalloxid ist.

7. Verfahren nach einem der Ansprüche 1 bis 6, **dadurch gekennzeichnet, dass** die Aktivmasse der Katalysatoren wenigstens ein Multimetalloxid der allgemeinen Formel I,
Mo₁₂BiₐFe_{b}X¹_{c}X²_{d}X³ₑX⁴_{f}Oₙ (I),
in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wölfram,
X⁴ = Silicium, Aluminium, Titan und/oder Zirkonium,
a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
ist.

8. Verfahren nach einem der Ansprüche 1 bis 7, **dadurch gekennzeichnet, dass** die Propenbelastung des Katalysatorfestbetts ≥ 90 Nl/l•h beträgt.

9. Verfahren nach einem der Ansprüche 1 bis 8, **dadurch gekennzeichnet, dass** die Propenbelastung des Katalysatorfestbetts ≥ 130 Nl/l•h beträgt.

10. Verfahren nach einem der Ansprüche 1 bis 9, **dadurch gekennzeichnet, dass** die Erhöhung der Temperatur des Katalysatorfestbetts über die Zeit so durchgeführt wird, dass der Umsatz des Propens bei einmaligem Durchgang des Reaktionsgasgemischs durch das Katalysatorfestbett 93 mol-% nicht unterschreitet.

11. Verfahren nach einem der Ansprüche 1 bis 9, **dadurch gekennzeichnet, dass** die Erhöhung der Temperatur des Katalysatorfestbetts über die Zeit so durchgeführt wird, dass der Propengehalt im Produktgasgemisch 10000 Gew.ppm nicht übersteigt.

12. Verfahren nach einem der Ansprüche 1 bis 11, **dadurch gekennzeichnet, dass** das Reaktionsgasausgangsgemisch 7 bis 15 Vol.-% Propen enthält.

## Claims

1. A process for the long-term operation of a heterogeneously catalyzed gas phase partial oxidation of propene to acrolein, by conducting a starting reaction gas mixture which comprises propene, molecular oxygen and at least one inert diluent gas through a fixed catalyst bed at elevated temperature whose catalysts are such that their active composition is at least one multimetal oxide which contains the elements molybdenum and/or tungsten and also at least one of the elements bismuth, tellurium, antimony, tin and copper, and by, in order to counteract the deactivation of the fixed catalyst bed, increasing the temperature of the fixed catalyst bed over the time, which comprises interrupting the gas phase partial oxidation at least once per calendar quarter and conducting a gas mixture G consisting of molecular oxygen, inert gas and optionally steam through the fixed catalyst bed at a temperature of the fixed catalyst bed of from 250 to 550°C.

2. The process according to claim 1, wherein the gas phase partial oxidation is interrupted at least once per calendar month and a gas mixture G consisting of molecular oxygen, inert gas and optionally steam is conducted through the fixed catalyst bed at a temperature of the fixed catalyst bed of from 250 to 550°C.

3. The process according to claim 1 or 2, wherein the period over which the gas mixture G is conducted through the fixed catalyst bed is from 2 h to 120 h.

4. The process according to any of claims 1 to 3, wherein the gas mixture G which is conducted through the fixed catalyst bed contains at least 4% by volume of oxygen.

5. The process according to any of claims 1 to 4, which is carried out in a tube bundle reactor.

6. The process according to any of claims 1 to 5, wherein the active composition of the catalysts is a multimetal oxide containing Mo, Bi and Fe.

7. The process according to any of claims 1 to 6, wherein the active composition of the catalysts is at least one multimetal oxide of the general formula I
Mo₁₂BiₐFe_{b}X¹_{c}X²_{d}X³ₑX⁴_{f}Oₙ (I)
in which the variables are each defined as follows:
X¹ = nickel and/or cobalt,
X² = thallium, an alkali metal and/or an alkaline earth metal,
X³ = zinc, phosphorus, arsenic, boron, antimony, tin, cerium, lead and/or tungsten,
X⁴ = silicon, aluminum, titanium and/or zirconium,
a = from 0.5 to 5,
b = from 0.01 to 5, preferably from 2 to 4,
c = from 0 to 10, preferably from 3 to 10,
d = from 0 to 2, preferably from 0.02 to 2,
e = from 0 to 8, preferably from 0 to 5,
f = from 0 to 10 and
n = a number which is determined by the valency and frequency of the elements in I other than oxygen.

8. The process according to any of claims 1 to 7, wherein the propene hourly space velocity on the fixed catalyst bed is ≥ 90 1 (STP)/l•h.

9. The process according to any of claims 1 to 8, wherein the propene hourly space velocity on the fixed catalyst bed is ≥ 130 1 (STP)/l•h.

10. The process according to any of claims 1 to 9, wherein the increase in the temperature of the fixed catalyst bed over time is carried out in such a way that the conversion of the propene in single pass of the reaction gas mixture through the fixed catalyst bed does not go below 93 mol%.

11. The process according to any of claims 1 to 9, wherein the increase in the temperature of the fixed catalyst bed over time is carried out in such a way that the propene content in the product gas mixture does not exceed 10 000 ppm by weight.

12. The process according to any of claims 1 to 11, wherein the starting reaction gas mixture contains from 7 to 15% by volume of propene.

## Revendications

1. Procédé d'exploitation à long terme d'une oxydation partielle en phase gazeuse sous catalyse hétérogène de propène en acroléine, selon lequel un mélange gazeux réactionnel de départ contenant du propène, de l'oxygène moléculaire et au moins un gaz diluant inerte traverse un lit catalytique fixe se trouvant à température élevée, dont les catalyseurs sont tels que leur masse active soit au moins un oxyde de plusieurs métaux qui contient les éléments molybdène et/ou tungstène, ainsi qu'au moins un des éléments bismuth, tellure, antimoine, étain et cuivre, et selon lequel, pour contrer la désactivation du lit catalytique fixe, la température du lit catalytique fixe est augmentée avec le temps, **caractérisé en ce que** l'oxydation partielle en phase gazeuse est interrompue au moins une fois par trimestre et un mélange gazeux G constitué d'oxygène moléculaire, de gaz inerte et éventuellement de vapeur d'eau traverse le lit catalytique fixe à une température du lit catalytique fixe de 250 à 550 °C.

2. Procédé selon la revendication 1, **caractérisé en ce que** l'oxydation partielle en phase gazeuse est interrompue au moins une fois par mois et un mélange gazeux G constitué d'oxygène moléculaire, de gaz inerte et éventuellement de vapeur d'eau traverse le lit catalytique fixe à une température du lit catalytique fixe de 250 à 550 °C.

3. Procédé selon la revendication 1 ou 2, **caractérisé en ce que** la durée pendant laquelle le mélange gazeux G traverse le lit catalytique fixe est de 2 h à 120 h.

4. Procédé selon l'une quelconque des revendications 1 à 3, **caractérisé en ce que** le mélange gazeux G qui traverse le lit catalytique fixe contient au moins 4 % en volume d'oxygène.

5. Procédé selon l'une quelconque des revendications 1 à 4, **caractérisé en ce qu'**il est réalisé dans un réacteur à faisceau de tubes.

6. Procédé selon l'une quelconque des revendications 1 à 5, **caractérisé en ce que** la masse active des catalyseurs est un oxyde de plusieurs métaux contenant Mo, Bi et Fe.

7. Procédé selon l'une quelconque des revendications 1 à 6, **caractérisé en ce que** la masse active des catalyseurs est au moins un oxyde de plusieurs métaux de formule générale I
Mo₁₂BiₐFe_{b}X¹_{c}X²_{d}X³ₑX⁴_{f}Oₙ (I)
dans laquelle les variables ont la signification suivante :
X¹ = nickel et/ou cobalt,
X² = thallium, un métal alcalin et/ou un métal alcalino-terreux,
X³ = zinc, phosphore, arsenic, bore, antimoine, étain, cérium, plomb et/ou tungstène,
X⁴ = silicium, aluminium, titane et/ou zirconium,
a = 0,5 à 5,
b = 0,01 à 5, de préférence 2 à 4,
c = 0 à 10, de préférence 3 à 10,
d = 0 à 2, de préférence 0,02 à 2,
e = 0 à 8, de préférence 0 à 5,
f = 0 à 10 et
n = un nombre déterminé par la valence et la fréquence des éléments différents de l'oxygène dans I.

8. Procédé selon l'une quelconque des revendications 1 à 7, **caractérisé en ce que** le chargement en propène du lit catalytique fixe est ≥ 90 Nl/l·h.

9. Procédé selon l'une quelconque des revendications 1 à 8, **caractérisé en ce que** le chargement en propène du lit catalytique fixe est ≥ 130 Nl/l·h.

10. Procédé selon l'une quelconque des revendications 1 à 9, **caractérisé en ce que** l'élévation de température du lit catalytique fixe avec le temps est réalisée de manière à ce que la conversion du propène lors d'un passage unique du mélange réactionnel gazeux au travers du lit catalytique fixe ne soit pas inférieure à 93 % en moles.

11. Procédé selon l'une quelconque des revendications 1 à 9, **caractérisé en ce que** l'élévation de température du lit catalytique fixe avec le temps est réalisée de manière à ce que la teneur en propène du mélange gazeux de produits ne dépasse pas 10 000 ppm en poids.

12. Procédé selon l'une quelconque des revendications 1 à 11, **caractérisé en ce que** le mélange réactionnel gazeux de départ contient 7 à 15 % en volume de propène.
